# EUROPÄISCHE PATENTSCHRIFT

(11) **EP 0 948 289 B1**
(45) Veröffentlichungstag und Bekanntmachung des Hinweises auf die Patenterteilung: **17.12.2003**
(21) Anmeldenummer: 97913165.3
(22) Anmeldetag: 20.10.1997
(51) Int. Cl.: A61B 18/00, A61F 9/008

(54) **VORRICHTUNG FÜR DIE BEHANDLUNG VON KÖRPERSUBSTANZEN**
DEVICE FOR TREATING BODILY SUBSTANCES
DISPOSITIF POUR TRAITER DES SUBSTANCES ORGANIQUES

(30) Priorität: 10.12.1996 DE 29621429 U; 08.04.1997 DE 19714476
(43) Veröffentlichungstag der Anmeldung: 13.10.1999
(73) Patentinhaber: Wavelight Laser Technologie AG, 91058 Erlangen (DE)
(72) Erfinder: DONITZKY, Christof, D-90542 Eckental (DE); PRIBBERNOW, Arnold, D-91245 Simmelsdorf (DE)
(74) Vertreter: von Hellfeld, Axel, Dr. Dipl.-Phys.
(86) Internationale Anmeldenummer: EP9705786
(87) Internationale Veröffentlichungsnummer: WO98025529

(56) Entgegenhaltungen:
- EP-A- 0 065 223
- EP-A- 0 164 751
- WO-A-95/17130
- US-A- 4 372 315
- US-A- 4 572 189
- US-A- 4 933 843
- US-A- 5 401 171
- US-A- 5 554 894

## Beschreibung

Die Erfindung betrifft eine Vorrichtung für die Behandlung von Körpersubstanzen mit einer Laserstrahlungsquelle, die gepulste Laserstrahlung mit Wellenlängen insbesondere im Infrarotbereich erzeugt, einer Einrichtung zur Führung der Laserstrahlung an den Behandlungsort, und mit einer Steuerung, mit der die Pulsenergie und/oder die Pulslänge und/oder die Pulsfrequenz einstellbar ist bzw. sind.

Ganz allgemein eignet sich die Erfindung für die Behandlung von Körpersubstanzen, d.h. Einwirkung auf Körpersubstanzen vielfältiger Art. Bei dem Begriff "Behandlung" kann es sich um eine Änderung des Zustandes dieser Substanzen handeln oder auch um ein Entfernen dieser Substanzen aus dem Körper oder eine Veränderung der Position der Substanzen im Körper. Die Körpersubstanzen können vielfältiger Art sein, zum Beispiel krankhaftes Gewebe oder eine krankhaft gebildete Substanz oder auch an sich gesundes Gewebe, welches für medizinische Zwecke entfernt werden soll. Nachfolgend wird die Erfindung beispielhaft mit Blick auf die sogenannte intraokulare Kataraktchirurgie näher beschrieben.

Ein Katarakt (Grauer Star) ist eine Veränderung der Linse des menschlichen Auges, die im sichtbaren optischen Bereich einen Transparenzverlust der Linse zur Folge hat. Es treten Trübungen im Bereich der Linse auf. Der Transparenzverlust bedingt eine Einschränkung der Sehfähigkeit. Die Kataraktchirurgie betrifft die operative Behandlung des Grauen Stars, bei der die getrübte Linse aus dem der optischen Wahrnehmung dienenden Strahlengang entfernt wird. In vergangenen Jahrhunderten erfolgte diese Entfernung durch den sogenannten Starstich, und es wurde anschliessend dem Patienten eine sogenannte Starbrille angepaßt. 1949 wurde erstmals durch den englischen Augenarzt H. Ridley im Anschluß an eine Kataraktoperation dem Patienten eine künstliche Augenlinse (PMMA) eingesetzt.

Vor dem Einsetzen einer intraokularen (neuen) Linse steht also bei der Kataraktchirurgie die Entfernung der getrübten ursprünglichen Linse.

Ein als immer wichtiger erkanntes Kriterium bei der Kataraktchirurgie ist die möglichst weitgehende Erhaltung angrenzender Gewebestrukturen bei der Entfernung der getrübten Linse.

Man unterscheidet bei der Operationstechnik die sogenannte intrakapsuläre Linsenentbindung und die extrakapsuläre Kataraktextraktion. Beim letztgenannten Verfahren ist das operative Ziel die Entfernung der getrübten Linse aus der Linsenkapsel, wobei die Linsenkapsel möglichst weitgehend in ihrer anatomischen Position erhalten bleiben soll.

In jüngster Zeit wurde als Beispiel einer extrakapsulären Katarakt-Operation die sogenannte Phakoemulsifikation entwickelt (W. Böke "Phakoemulsifikation. Warum?", Klin. Mbl. Augenheilk., 197 (1990) 100-105, F. Enke Verlag Stuttgart). Die Phakoemulsifikation ist im Prinzip ein klassischer chirurgischer Eingriff mit einem Schnitt.

In jüngerer Zeit wurde zur Verringerung von Gewebeirritationen durch Schnitte und anderen Gewebemanipulationen die sogenannte Ultraschall-Phakoemulsifikation in größerem Umfang eingesetzt (vgl. Jeffrey W. Berger, Jonathan H. Talamo, Kevin J. LaMarche, Seon-Ho Kim, Robert W. Snyder, Donald J. D'Amico, George Marcellino "Temperature Measuring During Phacoemulification and Erbium:YAG Laser Phacoablation in Model Systems", Journal of Cataract Refract Surg., Vol. 22, April 1996, S. 372 bis 378). Diese Ultraschall-Phakoemulsifikation erlaubt eine weitgehende Minimierung der interoperativen Belastungen sowie der postoperativen Komplikationen. Bei dieser Technik erfolgt die Entfernung des Linsenmaterials durch ein Saug-Spülsystem. Die Energie zur Zerteilung der Linse ist Ultraschall-Schwingung, die in den Linsenkörper eingebracht (eingekoppelt) wird.

Die Energie zur Zerteilung der getrübten Linse kann auch durch Laserstrahlung bereitgestellt werden (vgl. z.B. den vorstehenden Artikel von J. W. Berger et al). Bei der sogenannten Laserphakovaporisation mit zum Beispiel Er-YAG- oder Er-YSGG-Lasern wird das Linsenmaterial durch die hohe Absorption der Laserstrahlung im IR-Bereich in dem zu bearbeitenden Gewebe zerteilt, wobei mit der Zerteilung eine Gewebeablation oder eine Gewebetrennung einhergeht (Ray P. Gailitis, Scott W. Patterson, Mark A. Samuels, Kerry Hagen, Qiushi Ren, George O. Waring "Comparison of Laser Phacovaporization Using the Er-YAG and the Er-YSGG Laser", Arch Ophthalmol, Vol. 111, Mai 1993, S. 697-700).

Die vorliegende Erfindung betrifft die Entfernung einer getrübten Linse mit Laserstrahlung, insbesondere IR-Laserstrahlung, die von einem Festkörperlaser erzeugt wird, wie einem Er-YAG-Laser oder einem Er-YSGG-Laser.

Wie oben bereits ausgeführt ist, kommt es bei der Kataraktchirurgie wesentlich darauf an, die nicht entfernten Gewebestrukturen möglichst weitgehend geschont zu belassen und Gewebeirritationen soweit als möglich zu vermeiden. Das Linsenmaterial unterscheidet sich aber sehr stark von Patient zu Patient und läßt sich nicht immer vom Chirurgen im voraus zutreffend hinsichtlich seines Verhaltens in bezug auf die Absorption und Wirkung der Laserstrahlung einschätzen. Hinzu kommt als weitere Schwierigkeit bei der kataraktchirurgie, daß das Material auch innerhalb einer zu entfernenden Linse stark inhomogene optische Eigenschaften aufweisen kann, es sich also je nach dem Ort, an dem die Laserstrahlung einwirkt, unterschiedlich hinsichtlich Absorption und Ablation verhalten kann.

Die vorstehend skizzierten Probleme treten nicht nur bei der Kataraktchirurgie auf, sondern allgemein bei der Behandlung von Substanzen des menschlichen Körpers ("Körpersubstanzen") mit Laserstrahlung. Bei der Behandlung von Körpersubstanzen mit Laserstrahlung kommt es regelmäßig darauf an, nur die kranken oder veränderten Bereiche mit der Strahlung zu behandeln, während mehr oder weniger eng benachbarte Bereiche möglichst nicht der Strahlung ausgesetzt werden sollen.

Die US-A-4 572 189 beschreibt eine elektronische Steuerung für ein chirurgisches Lasersystem, bei dem lediglich die maximale Pulsanzahl sowie zwei Pulsdauern vorwählbar sind. Die US-A4 933 843 und die EP-A-0164751 beschreiben chirurgische Lasersysteme, bei denen eine Menuesteuerung erlaubt, mangelhafte Einstellungen des Systems zu erkennen.

Bei Anwendung in der Kataraktchirurgie betrifft die Erfindung die sich aus dem unterschiedlichen Linsenmaterial bei verschiedenen Patienten und die durch die Inhomogenität der optischen Eigenschaften innerhalb einer Linse ergebenden technischen Probleme, und es ist Ziel der Erfindung, eine Vorrichtung für die intraokulare Kataraktchirurgie der eingangs genannten Art so auszugestalten, daß dem Chirurgen eine Hilfe an die Hand gegeben wird, mit der er die intraokulare Applikation der Laserenergie so steuern kann, daß eine saubere Entfernung der getrübten Linse ohne Beeinträchtigung benachbarter Gewebestrukturen weitgehend gefördert ist.

Erfindungsgemäß wird diese Aufgabe bei einer Vorrichtung für die intraokulare Kataraktchirurgie der eingangs genannten Art dadurch gelöst, daß die Steuerung eine erste Einrichtung aufweist, mit der für die Pulsenergie und/oder die Pulslänge und/oder die Pulsfrequenz vor einer Operation ein zulässiger Bereich vorgebbar ist, und daß die Steuerung eine zweite Einrichtung aufweist, mit der die Pulsenergie und/oder die Pulslänge und/oder die Pulsfrequenz während der Operation auf einen Wert bzw. Werte in dem vorgegebenen Bereich einstellbar ist bzw. sind.

Die Erfindung eignet sich insbesondere auch für die Verwirklichung in Geräten für die Dermatologie, insbesondere für die Behandlung von Narben oder Falten. Bei Einsatz in der Dermatologie kann als Führungseinrichtung Fasermaterial oder auch ein als solches bekannter Spiegelgelenkarm dienen, d.h. die Strahlung wird über Spiegel an den gewünschten Ort geführt.

Weiterhin eignet sich die Erfindung auch für Geräte in der Dentalogie, insbesondere für die Behandlung von Zahnhartsubstanzen, wie Schmelz und Dentin.

Bei Einsatz in der Zahnheilkunde eignet sich insbesondere ein Er:YAG-Festkörperlaser als Laserstrahlungsquelle, und zwar bevorzugt sogar in Kombination mit einem weiteren Laser, wie einem Nd:YAG-Festkörperlaser (Wellenlänge: 1064 nm). Die beiden Laser werden bevorzugt alternierend über zwei verschiedene Führungseinrichtungen eingesetzt, wobei aber die gleiche erfindungsgemäße Steuerung für die Pulsenergie, Pulslänge bzw. Pulsfrequenz vorgesehen wird.

Durch die Erfindung ist es dem Operateur möglich, die eingekoppelte Laserstrahlungsenergie in einfacher Weise zum Beispiel dann deutlich zu verringern, wenn sich der Ort der Einkoppelung der Energie einem kritischen Bereich nähert, zum Beispiel einer benachbarten, möglichst zu erhaltenden Gewebestruktur, wie dem Kapselsack. Die Erfindung ermöglicht also auf der einen Seite die Möglichkeit der Einstellung der jeweils momentan während der Operation eingebrachten Laserstrahlungsenergie in Abhängigkeit vom Ort, an dem die Laserstrahlung wirkt, und in Abhängigkeit davon, wie weit die Operation fortgeschritten ist, und zum anderen ermöglicht die Erfindung auch die Begrenzung der überhaupt einstellbaren Pulsenergie und/oder Pulslänge und/oder Pulsfrequenz, so daß während der Operation gesichert ist, daß nicht (z.B. versehentlich) zuviel Energie oder Strahlung mit unerwünschter Pulslänge und/oder Pulsfrequenz eingekoppelt wird.

Die Erfindung ermöglicht also einen effizienten (insbesondere schnellen) und kontrollierten Operationsverlauf mit gesteuerter und überwachter Energieeinbringung, wobei der Chirurg vorab durch Einschätzung des jeweils zu bearbeitenden Materials, z.B. des Linsenmaterials, einen zulässigen Bereich für die überhaupt einstellbare Pulsenergie und/oder Pulsfrequenz und/oder Pulslänge vorgeben kann, der für das zu bearbeitende Material optimal erscheint. Während der Operation (interoperativ) kann der Chirurg mit einem einfachen Handhabungssystem, wie zum Beispiel einem mit dem Fuß betätigbaren Einstellelement oder auch einem mit der Hand einstellbaren Einstellelement, die gewünschten Parameter (Pulsenergie, Pulslänge oder Pulsfrequenz) momentan ändern, je nach dem Operationsfortschritt und dem gerade bearbeiteten Material sowie dem Ort der Strahlungseinwirkung. Dies erlaubt dem Chirurgen eine deutlich verbesserte Reaktion auf individuelle Gewebeunterschiede in der Linse während der Operation sowie beim Erreichen von angrenzenden, zu erhaltenden Gewebestrukturen.

Vorteilhafte Ausgestaltungen der Erfindung sind in den abhängigen Ansprüchen beschrieben.

Nachfolgend wird ein Ausführungsbeispiel der Erfindung in der intraokularen Kataraktchirurgie anhand der Zeichnung näher erläutert. Es zeigt:
- Fig. 1: schematisch eine Vorrichtung für die intraokulare Kataraktchirurgie und
- Fig. 2: schematisch eine Steuerung für die intraokulare Kataraktchirurgie.

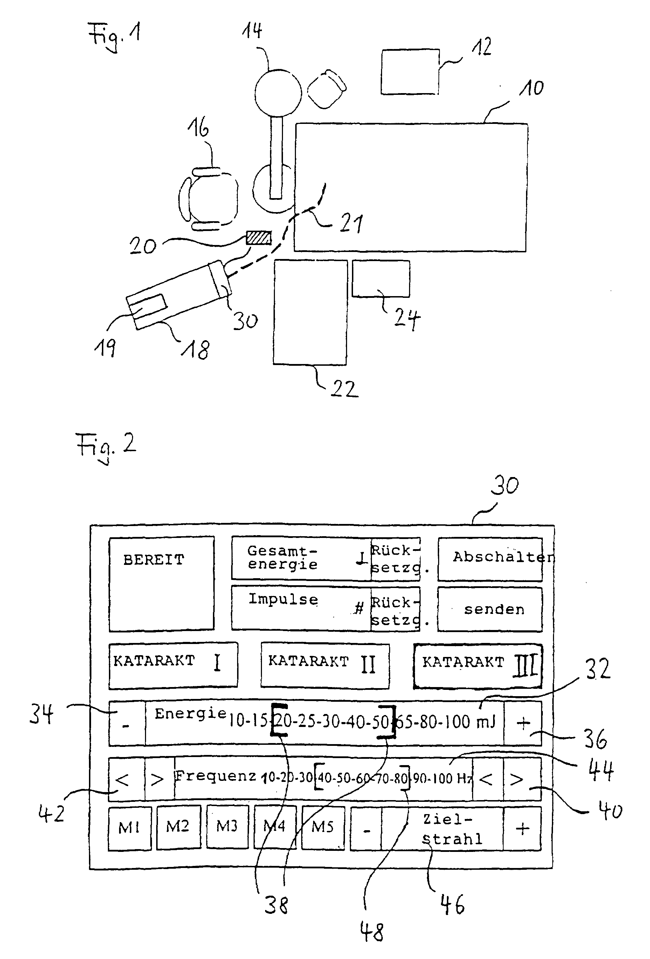

Gemäß Fig. 1 wird ein Patient auf einer Patientenliege 10 plaziert. Ein Narkose- und Diagnose-System sind mit dem Bezugszeichen 12 schematisch angedeutet, ebenso ein OPMI (Operations-Mikroskop) mit dem Bezugszeichen 14. Der Operateur sitzt auf einem Stuhl 16. Eine Laserstrahlungsquelle 19 ist in einem Phako-Ablationssystem 18 angeordnet. Als Laserstrahlungsquelle 19 dient ein Erbium YAG-Laser. Mittels einer Faseroptik 21 wird die Ausgangsstrahlung des Lasers zum Auge des Patienten übertragen. Diese Technik entspricht insoweit dem Stand der Technik und braucht hier nicht weiter erläutert zu werden.

Der Operateur kann mit einem Fußtaster 20 wahlweise die Pulsenergie und/oder die Pulslänge und/oder insbesondere die Pulsfrequenz (Pulsfolgefrequenz) einstellen.

Ein Saug-/Spülsystem 22 zur Entfernung der Linsenreste ist in Fig. 1 gezeigt. Neben dem Saug-/Spülsystem 22 ist ein Monitor 24 (zum Beispiel auch ein Video) für den Operateur angeordnet.

Von der Steuerung 30, welche im Phako-Ablationssystem 18 integriert ist, interessieren hier insbesondere die in Fig. 2 mit Bezugszeichen 32 bis 48 versehenen Tastenfelder und Funktionen. Ein Feld 32 zeigt verschiedene Energiewerte für die einstellbare Pulsenergie, nämlich 10 mJ, 15 mJ etc. bis 100 mJ (Pulsenergie). Über eine Tastatur (nicht näher dargestellt) können im "Energiefeld" 32 obere und untere Grenzen für einstellbare Pulsenergien vor einer Operation vorgegeben werden oder auch während einer Operation geändert werden. Diese Grenzen sind in Fig. 2 mit eckigen Klammern 38 angedeutet. Die Klammern 38 grenzen also zulässige einstellbare Pulsenergiewerte ein, beim dargestellten Ausführungsbeispiel gemäß Fig. 2 kann also der Operateur zwanzig bis fünfzig mJ Pulsenergie einstellen. Eine Änderung der Pulsenergie während der Operation kann zum Beispiel mit den beiden Tasten 34, 36 erfolgen. Stellt der Rechner nach Festlegung der Grenzwerte 38 den minimalen Wert der Pulsenergie (20 mJ) ein, so kann der Operateur durch Drücken der "+"-Taste 36 die Pulsenergie schrittweise auf 25 mJ, 30 mJ etc. erhöhen, wobei der jeweils gültige Wert der Pulsenergie zum Beispiel durch starkes Aufleuchten des jeweils eingestellten Pulsenergiewertes hervorgehoben ist.

Im Feld 44 der Steuerung 30 sind einstellbare Pulsfolgefrequenzen dargestellt, wobei der Operateur Frequenzen von 10 bis 100 Hz in Schritten einstellen kann. Die Einstellung erfolgt mit den Tasten 40 bzw. 42 (Erhöhung bzw. Verringerung der Frequenz). Wahlweise kann die Vorrichtung auch so ausgestaltet sein, daß alternativ oder zusätzlich zu dem vor der Operation einstellbaren Bereich der Pulsenergie (Bereichsschranken 38) auch die Pulsfolgefrequenz vorab hinsichtlich der überhaupt zugänglichen Frequenzen einstellbar ist, was in Fig. 2 durch die Klammer 48 angedeutet ist, die analog der oben beschriebenen Klammer 38 hinsichtlich der Pulsenergie wirkt.

Die Vorrichtung weist einen sogenannten Zielstrahl (aiming beam) auf, der dem Operateur die Position des nicht sichtbaren IR-Laserstrahl anzeigt. Als Zielstrahl kann zum Beispiel der von einer Laserdiode abgegebene Strahl im sichtbaren Bereich dienen (z.B. 635 nm). Die Laserdiode für den Zielstrahl ist im Phako-Ablationssystem 18 untergebracht und die Strahlung wird ebenfalls über die Faseroptik 21 übertragen.

## Patentansprüche

1. Vorrichtung für die Behandlung von Körpersubstanzen mit einer Laserstrahlungsquelle (19), die gepulste Laserstrahlung erzeugt, einer Einrichtung (21) zur Führung der Laserstrahlung an den Behandlungsort, und mit einer Steuerung (30), mit der die Pulsenergie und/oder die Pulslänge und/oder die Pulsfrequenz einstellbar ist bzw. sind,
**dadurch gekennzeichnet, daß**
die Steuerung (30) eine erste Einrichtung (38, 48) aufweist, mit der für die Pulsenergie und/oder die Pulslänge und/oder die Pulsfrequenz vor einer Operation ein zulässiger Bereich vorgebbar ist, und daß die Steuerung (30) eine zweite Einrichtung (20) aufweist, mit der die Pulsenergie und/oder die Pulslänge und/ oder die Pulsfrequenz während der Operation auf einen Wert bzw. Werte in dem vorgegebenen Bereich einstellbar ist bzw. sind.

2. Vorrichtung nach Anspruch 1,
**dadurch gekennzeichnet, daß**
mittels der Steuerung (30) ein zulässiger Bereich (38) für die Pulsenergie vorgebbar ist und daß während der Operation ein Wert in diesem vorgegebenen Bereich wahlweise einstellbar ist.

3. Vorrichtung nach einem der Ansprüche 1 oder 2,
**dadurch gekennzeichnet, daß**
durch die Steuerung ein zulässiger Bereich für die Pulsfolgefrequenz vorgebbar ist und daß während der Operation ein Wert in diesem vorgegebenen Bereich wahlweise einstellbar ist.

4. Vorrichtung nach einem der vorhergehenden Ansprüche,
**dadurch gekennzeichnet, daß**
die Laserstrahlungsquelle Laserstrahlung im Infrarotbereich, insbesondere im mittleren Infrarotbereich erzeugt, und insbesondere eine mit Erbium dotierte Festkörperlaserquelle, wie eine Erbium-YAG-Laserquelle, ist.

5. Vorrichtung nach einem der vorhergehenden Ansprüche,
**dadurch gekennzeichnet, daß**
die zweite Einrichtung, mit der die Pulsenergie und/oder die Pulslänge und/oder die Pulsfrequenz während der Operation einstellbar ist, ein vom Operateur bedienbarer Fußschalter (20) ist.

6. Vorrichtung nach einem der vorhergehenden Ansprüche für die intraokulare Kataraktchirurgie,
**dadurch gekennzeichnet, daß**
die Führungseinrichtung (21) so gestaltet ist, daß die Laserstrahlung in das Okulargewebe einkoppelbar ist.

7. Vorrichtung nach der Ansprüche 1 bis 5,
**dadurch gekennzeichnet, daß**
die Führungseinrichtung so gestaltet ist, daß die Laserstrahlung auf Zahnmaterial aufbringbar ist.

8. Vorrichtung nach einem der Ansprüche 1 bis 5,
**dadurch gekennzeichnet, daß**
mittels der Führungseinrichtung die Laserstrahlung auf die Haut aufbringbar ist, wobei für die Führungseinrichtung insbesondere Fasermaterial oder ein Spiegelgelenkarm mit Spiegeln vorgesehen ist.

## Claims

1. A device for treating bodily substances, comprising a laser beam source (19) which produces pulsed laser radiation, an arrangement (21) for guiding the laser beam to the location of treatment, and further comprising a control device (30) by means of which the pulse energy and/or pulse length and/or pulse frequency can be adjusted,
**characterised in that**
said control device (30) comprises a first arrangement (38, 48) by means of which an admissible range for the pulse energy and/or pulse length and/or pulse frequency can be predetermined before an operation, and that said control device (30) comprising a second arrangement (20) by means of which the pulse energy and/or pulse length and/or pulse frequency can be adjusted to a given value or values within the predetermined range during the operation.

2. The device according to claim 1,
**characterised in that**
an admissible range (38) can be predetermined for the pulse energy by means of the control device (30) and **in that** a value within said predetermined range may optionally be adjusted during the operation.

3. The device according to any of claims 1 or 2,
**characterised in that**
an admissible range can be predetermined for the pulse repetition frequency by means of the control device and **in that** a value within said predetermined range may optionally be adjusted during the operation.

4. The device according to one of the preceding claims,
**characterised in that**
laser radiation is produced by the laser beam source in the infrared range, in particular in the middle infrared range, and particularly is a solid-state laser source doped with erbium, such as an erbium YAG laser source.

5. The device according to one of the preceding claims,
**characterised in that**
said second arrangement by which the pulse energy and/or pulse length and/or pulse frequency can be adjusted during the operation is a foot switch (20) which can be actuated by the surgeon.

6. The device according to any of the preceding claims for intraocular cataract surgery,
**characterised In that**
the guide means (21) is designed in such a manner that the laser radiation can be applied to the ocular tissue.

7. The device according to claims 1 to 5,
**characterised in that**
the guide means is designed in such a manner that the laser radiation can be applied to tooth material.

8. The device according to one of claims 1 to 5,
**characterised in that**
the laser radiation can be applied to the skin by means of the guide means, wherein fibre material or an articulated arm with mirror is provided for said guide means.

## Revendications

1. Dispositif pour le traitement de substances du corps humain équipé d'une source de rayonnement laser (19), qui génère un rayonnement laser pulsé, d'un appareil (29) pour le guidage du rayonnement laser au lieu de traitement, et d'une commande (30), avec laquelle l'énergie d'impulsion et/ou la longueur d'impulsion et/ou la fréquence d'impulsion peut ou peuvent être réglée(s), **caractérisé en ce que** la commande (30) présente un premier appareil (38, 48) qui permet de prédéfinir une plage autorisée pour l'énergie d'impulsion et/ou la longueur d'impulsion et/ou la fréquence d'impulsion d'une opération et **en ce que** la commande (30) présente un second appareil (20) qui permet de régler l'énergie d'impulsion et/ou la longueur d'impulsion et/ou la fréquence d'impulsion pendant l'opération sur une valeur ou des valeurs dans la plage prédéfinie.

2. Dispositif selon la revendication 1, **caractérisé en ce qu'**une plage (38) autorisée est prédéfinie pour l'énergie d'impulsion au moyen de la commande (30) et **en ce qu'**une valeur peut être réglée au choix dans cette plage prédéfinie pendant l'opération.

3. Dispositif selon l'une quelconque des revendications 1 ou 2, **caractérisé en ce qu'**une plage autorisée pour la fréquence de succession d'impulsions peut être prédéfinie par la commande et **en ce qu'**une valeur peut être réglée au choix dans cette plage prédéfinie pendant l'opération.

4. Dispositif selon l'une quelconque des revendications précédentes, **caractérisé en ce que** la source de rayonnement laser génère un rayonnement laser dans la plage infrarouge, en particulier dans la plage infrarouge moyenne, et est en particulier une source laser à corps solide dopée avec de l'erbium, par exemple une source laser YAG à erbium.

5. Dispositif selon l'une quelconque des revendications précédentes, **caractérisé en ce que** le second appareil, qui permet de régler l'énergie d'impulsion et/ou la longueur d'impulsion et/ou la fréquence d'impulsion pendant l'opération, est un interrupteur à pied (20) pouvant être commandé par l'opérateur.

6. Dispositif selon l'une quelconque des revendications précédentes pour la chirurgie de la cataracte intra-oculaire, **caractérisé en ce que** l'appareil de guidage (21) est conçu de telle sorte que le rayonnement laser peut être injecté dans le tissu oculaire.

7. Dispositif selon les revendications 1 à 5, **caractérisé en ce que** l'appareil de guidage est conçu de telle sorte que le rayonnement laser peut être appliqué sur du matériau de dent.

8. Dispositif selon l'une quelconque des revendications 1 à 5, **caractérisé en ce que** le rayonnement laser peut être appliqué sur la peau au moyen de l'appareil de guidage, en particulier du matériau de fibre ou un bras articulé à miroir avec des miroirs étant prévu pour l'appareil de guidage.
